# EUROPÄISCHE PATENTSCHRIFT

(11) **EP 2 263 794 B1**
(45) Veröffentlichungstag und Bekanntmachung des Hinweises auf die Patenterteilung: **28.03.2012**
(21) Anmeldenummer: 10005448.5
(22) Anmeldetag: 26.05.2010
(51) Int. Cl.: B01J 31/40, B01J 31/10, B01J 39/20, B01J 49/00, C07C 37/20, C07C 37/82, C07C 39/16

(54) **Regeneration von sauren Ionenaustauschern**
Regeneration of acid ion exchangers
Régénération d'échangeurs d'ions acides

(30) Priorität: 30.05.2009 DE 102009023551
(43) Veröffentlichungstag der Anmeldung: 22.12.2010
(73) Patentinhaber: Bayer MaterialScience AG, 51368 Leverkusen (DE)
(72) Erfinder: Prein, Michael, Dr., 47809 Krefeld (DE); Münnich, Christian, Dr., 51377 Leverkusen (DE); Blaschke, Ulrich, Dr., 47829 Krefeld (DE)

(56) Entgegenhaltungen:
- DE-A1- 2 727 866
- DE-A1- 19 956 229
- GB-A- 842 209
- US-A- 3 242 219

## Beschreibung

Gegenstand der Erfindung ist ein Verfahren zur Regenerierung von sauren Kationenaustauschern, die insbesondere als Katalysatoren bei der Umsetzung von Phenolen mit Aldehyden oder Ketonen zu Bisphenolen, insbesondere zu Bisphenol-A, verwendet werden, mit Säuren mit der Maßgabe, dass diese Kationenaustauscher bei dem erfindungsgemäßen Verfahren geringste mechanische Beschädigung infolge von Quellvorgängen beim Regenerierungsprozess erfahren.

Kondensationsreaktionen von Phenolen mit Carbonylverbindungen zu Bisphenolen unter Verwendung saurer Katalysatoren, insbesondere unter Verwendung von Kationenaustauschern sind bekannt (siehe beispielsweise GB 842 209 A, GB 849 565 A oder GB 883 391 A). Es ist ebenfalls bekannt, saure Kationenaustauscher, die durch Metallionen deaktiviert wurden oder durch organische Nebenprodukte aus der Bisphenol-Herstellung verschmutzt wurden ("fouling"), durch geeignete Maßnahmen zu regenerieren. So wird z.B. in der EP 324 080 A ein Verfahren zur Regenerierung von sauren Kationenaustauschern durch Behandlung mit Basen und Säuren beschrieben. Allerdings wird der Aspekt der mechanischen Unversehrtheit der Kationenaustauscher hier überhaupt nicht betrachtet und es gibt auch keine Hinweise, wie ein solches Ziel zu erreichen ist. Darüber hinaus ist die Abfolge der verschiedenen Wäschen des Ionenaustauschers mit Wasser, Base und Säure in der Praxis so aufwändig, dass sie für eine großtechnische Produktion, insbesondere für die Regenerierung in einer bestehenden Anlage praktisch nicht infrage kommt.

Auch in der Veröffentlichung RD 369008 (Database Document Number) von "Research and Disclosure" (Derwent Publications Ltd., London, GB, Database Accession Number1995-080031) wird von der Regenerierung saurer Kationenaustauscher berichtet, wobei eine aufwändige Abfolge von mehreren Behandlungen mit verschiedenen Agentien, wie Wasser, Keton, Base und Säure empfohlen wird, die ungeeignet für eine Regenerierung im technischen Maßstab in einer bestehenden kontinuierlich arbeitenden Produktionsanlage ist, da zu viele Fremdprodukte in den Herstellprozess eingetragen und wieder sicher entfernt werden müssen. Auch hier werden die Folgen einer mechanischen Beschädigung des Kationenaustauschers durch Quellvorgänge nicht betrachtet.

In DE 2 727 866 A1 wird eine verhältnismäßig einfache Abfolge von Wäschen mit Phenol, Wasser und Säure unter Verzicht auf eine Wäsche mit Basen beschrieben. Allerdings werden auch hier keine Angaben zur Quellung der Kationenaustauscher mit Wasser und den möglichen Folgen einer mechanischen Zerstörung gemacht. Angaben über die Auswirkungen der Wasserkonzentration beziehungsweise über Konzentrationsobergrenzen von Wasser im Spülmedium Phenol und Wasser beziehungsweise Phenol, Wasser und Säure auf die Eigenschaften des so behandelten Ionenaustauschers fehlen.

Im Stand der Technik werden keine definierte Obergrenzen einer zulässigen Wasserkonzentration im Phenol-Säure-Gemisch bei der Regenerierung festgelegt, bei deren Einhaltung ein übermäßiges Quellen und damit ein Bersten des Kationenaustauscherkorns weitestgehend vermieden werden kann. Im Gegenteil, nach den Angaben in der DE 2 727 866 A1 auf Seite 8, kann der Wasseranteil für eine erfolgreiche Regenerierung des lonenaustauscherbetts bis zu 30 Gew% betragen. Unter solchen Bedingungen besteht allerdings größte Gefahr für ein übermäßiges Quellen des Kationenaustauschers mit den genannten Folgen. Somit ist die angegebene Obergrenze nicht geeignet um die hier dargestellte Aufgabe zu lösen.

Dieser Aspekt ist deswegen von großer Bedeutung, da Kationenaustauscher, die üblicherweise auch durch "fouling" deaktiviert sind - d.h. durch Einlagerung von höhermolekularen Kondensationsprodukten z.B. aus Phenol und Aceton in das Polymernetzwerk des Katalysatorkorns - bei der Behandlung mit zu hohen Konzentrationen von Wasser so stark quellen können, dass der osmotische Druck in dem mit diesen "fouling"-Komponenten belasteten Ionenaustauscherkorn so hoch werden kann, dass es zerreißt. Dadurch werden Feinanteile des Ionenaustauscherkorns gebildet, die im bestimmungsgemäßen Betrieb nicht nur zur Verstopfung von Festbettreaktoren und Filtern führen, sondern auch in nachgeschalteten Prozessstufen große Störungen durch Nebenreaktionen mit Qualitätsproblemen als Folge davon hervorrufen können.

Die Kationenaustauscher werden in der Regel als durchströmte Kornschüttungen verwendet. Durch die Kornbruch bedingte Veränderung der Gesamtkorngrößenverteilung des Kationenaustauschers kann weiterhin die Druckverlustcharakteristik in der durchströmten Kationenaustauscherschüttung negativ beeinflusst werden, was zu höherem Druckverlust in der Schüttung und möglichen hydraulischen Limitierungen und somit Produktionsmengenlimitierungen führt.

Es bestand daher die Aufgabe, ein technisch einfaches und kostengünstiges Verfahren zur Regenerierung von deaktivierten sauren Kationenaustauschern zu finden, das die Kornstruktur des Ionenaustauscherkoms weitgehend intakt lässt und die an der Polymermatrix gebundenen funktionellen sauren Gruppen, wie z.B. Sulfonsäuregruppen, weitgehend wieder in ihre acide Form überführt.

Die Vermeidung von so ausgelösten mechanischen Beschädigungen ist insofern von großer Bedeutung, da feinste Bruchstücke von Körnern des Kationenaustauschers nach dessen Regenerierung und in dessen bestimmungsgemäßen Betrieb erhebliche Probleme z.B. durch unerwünschte Nebenreaktionen, Verstopfungen von Anlagenteilen oder Druckverlustlimitierungen bewirken können. Das erfindungsgemäße Verfahren wird angewendet auf saure Kationenaustauscher, die in chemischen Reaktionen als Katalysatoren verwendet werden, und die im Laufe ihres Einsatzes dort einen Teil ihrer katalytischen Aktivität verloren haben, so dass der Reaktionsumsatz unzureichend ist. Durch das erfindungsgemäße Verfahren der Regenerierung von derartig deaktivierten sauren Kationenaustauschem mit Säuren wird deren katalytische Aktivität wieder so erhöht, dass eine kostengünstige mehrfache Wiederverwendung dieser Kationenaustauscher in chemischen Synthesen ermöglicht wird. Erfindungsgemäß regenerierte Ionenaustauscher sind insbesondere saure, ggf. auch modifizierte Kationenaustauscher, die als Katalysatoren bei der Umsetzung von Phenolen mit Aldehyden oder Ketonen zu Bisphenolen, insbesondere zu Bisphenol-A verwendet werden. Modifizierte Kationenaustauscher sind z.B. Kationenaustauscher, die zusätzlich zu ihrer Funktion als Kationenaustauscher durch z.B. acide Gruppen durch Aufbringen weitere chemisch aktiven Komponenten mit zusätzlichen Funktionen ausgerüstet werden. Solche chemisch aktiven Komponenten können z.B. Cokatalysatoren sein, die z.B. u.a. Thiolfunktionen aufweisen.

Überraschenderweise wurde nun gefunden, dass die erfindungsgemäße Aufgabe besonders effizient gelöst wird, wenn deaktivierte oder partiell deaktivierte saure Kationenaustauscher einem Regenerierungsprozess unterzogen werden, der folgende Schritte umfasst:
a) Spülen eines außer Betrieb genommenen Ionenaustauscher-Katalysators mit einer Mischung aus Phenol und Säure, wobei der Gehalt an Säure in der Phenol-Säure-Mischung kleiner 10 Gew.-% und größer 0 Gew.-%, vorzugsweise 3 - 5 Gew.-% ist. Die Temperatur in dem mit der Phenol-Säure-Mischung gefluteten Reaktorbett geht dabei von 45°C bis 90°C, bevorzugt von 60°C bis 70°C. Bevorzugt wird eine Menge an Phenol-Säure-Mischung beträgt 1 bis 6 Bettvolumina, besonders bevorzugt 2 bis 4 Bettvolumina (BV) entsprechend dem Ionenaustauscher-Katalysatorbett im Reaktor.
b) Gradientenweise Erhöhung des Wassergehaltes in der zur Spülung des Ionenaustauscher-Katalysator verwendeten Phenol-Säure-Mischung von 0 Gew.-% Wasser auf 1 Gew.-% Wasser, wobei das Gesamtvolumen dieser Mischung als Spüllösung bevorzugt 0,25 bis 4 BV beträgt und gradientenweise Erhöhung des Wassergehaltes in der zur Spülung des Ionenaustauscher-Katalysator verwendeten Phenol-Säure-Mischung von 1 Gew.-% Wasser auf 5 Gew.-% Wasser, wobei das Gesamtvolumen dieser Mischung als Spüllösung bevorzugt 0,25 bis 4 BV beträgt.
c) Spülen des Ionenaustauscher-Katalysators mit einer Mischung aus Phenol und Wasser, die bevorzugt 5 - 25 Gew.-% Wasser enthält, solange, bis die elektrische Leitfähigkeit der Spüllösung kleiner 50 µS/cm, vorzugsweise kleiner 20 µS/cm ist.
d) Entwässern des so gespülten Ionenaustauscher-Katalysators mit reinem Phenol, solange, bis der Restwassergehalt in dem Spül-Phenol kleiner als 1 Gew.-% ist.

Gradientenweise Erhöhung bezeichnet dabei die Erhöhung des Gehalts einer Komponente in der Lösung, die stufenweise, linear oder nicht linear erfolgen kann. Bevorzugt wird die Erhöhung des Gehalts einer Komponente mit konstanter Rate (z.B. + 1 % pro BV Spüllösung) durchgeführt.

Die Regeneration kann sowohl direkt im Reaktor oder außerhalb des Reaktors stattfinden. Bevorzugt wird die Regeneration im Reaktor durchgeführt.

Saure Kationenaustauscher im Sinne der Erfindung sind beispielsweise Ionenaustauscher, die als Polymermatrix ein teilvernetztes Polystyrol, zum Beispiel mit Divinylbenzol vernetztes Polystyrol, enthalten, mit Vernetzungsgraden von 1 bis 20%, vorzugsweise 1 bis 10%, und die an die Polymermatrix chemisch gebundene Sulfonsäuregruppen in der H-Form (oder aciden Form) aufweisen. Typische Sulfonsäurekonzentrationen der sauren Kationenaustauscher liegen beispielsweise im Bereich von 3 bis 7 mol / kg Trockenmasse des Kationenaustauschers. Diese Ionenaustauscher können gegebenenfalls weitere kovalent oder ionisch gebundene Cokatalysatoren chemisch gebunden enthalten; sie sind makroporös oder gelförmig, wie es in US-A-4,191,843 und US-A-3,037,052 beschrieben ist, und weisen eine feinteilige Kugelform auf.

Derartige saure Kationenaustauscher sind wichtige Katalysatoren für die Herstellung von Bisphenolen aus Ketonen oder Aldehyden und Phenolen. Die erfindungsgemäß regenerierten sauren Ionenaustauscher werden beispielsweise als Katalysatoren im wirtschaftlich bedeutsamen Herstellverfahren für Bisphenol-A (BPA) aus Aceton und Phenol verwendet. Die in einem solchen Verfahren eingesetzten Ionenaustauscher verlieren im Laufe ihres Gebrauchs, im Allgemeinen nach einem oder mehreren Jahren, langsam ihre katalytische Aktivität. Gründe für diese Deaktivierung sind beispielsweise:
- Ionenaustausch an den Sulfonsäuregruppen durch Metall-Ionen, die zum Beispiel durch die eingesetzten Rohstoffe in den Herstellprozess eingeführt werden. Die dabei entstehenden Metall-Sulfonatgruppen sind nicht mehr katalytisch aktiv.
- Einlagerung von höhermolekularen Verbindungen in das Polymernetzwerk des Ionenaustauscherkorns; sog. "fouling". Diese höhermolekularen Verbindungen, die auch teerartigen Charakter annehmen können, verhindern den Zugang der Reaktionsteilnehmer zu den aktiven Zentren im Katalysatorkorn. Sie stellen im Wesentlichen höhermolekulare Kondensationsprodukte aus Phenol, Bisphenol, Aceton oder Acetonderivaten dar.

Wesentliche Schritte des erfindungsgemäßen Regenerierungsprozesses sind daher beispielsweise
a) die Behandlung mit Säuren, wobei einerseits die Metallsulfonat-Gruppen (Me-Form) am Ionenaustauscherharz in Sulfonsäure-Gruppen (H-Form) überführt werden, und wobei andererseits die oben beschriebenen höhermolekularen "fouling"-Komponenten in niedermolekulare Bestandteile sauer gespalten werden; und
b) die der Säure-Behandlung nachfolgende Behandlung mit definierten Mengen an Wasser, um das Ionenaustauscherkorn so langsam und so hinreichend wie nötig zu quellen, so dass einerseits die niedermolekularen Stoffe aus dem Ionenaustauscherkorn hinreichend ausgewaschen und die aktiven Zentren wieder frei gelegt werden können, und dass andererseits mechanische Beschädigungen am Kationenaustauscherkorn weitgehend ausgeschlossen werden.

Das Freispülen von Säureresten und das Entwässern sind bereits bekannte Behandlungsprozeduren an Kationenaustauschern und sind dem Fachmann hinreichend bekannt und sind beispielsweise in WO 2001037992 A1 beschrieben.

In einer bevorzugten Ausfühungsform verbleibt der Ionenaustauscher-Katalysator während des Regenerierungsprozesses im Reaktor, der außer Betrieb genommen wird. Die Säuren und das Wasser werden bevorzugt über getrennte Leitungen in den phenolischen Hauptstrom zugeführt und z.B. über statische Mischer eingemischt. In einer anderen Ausführungsform des erfindungsgemäßen Verfahrens werden die Lösungen in einem separaten Gefäß vorgemischt und dem zu regenierenden Kationenaustauscher zugeführt. Die abfließenden Spülflüssigkeiten werden aus dem üblichen Herstell-Prozess ausgeschleust und separat aufgearbeitet.

Die schonende Regenerierung des Kationenaustauschers kann auch außerhalb des Produktionsreaktors durchgeführt werden. Hierzu muss der Kationenaustauscher aus dem Reaktor in ein geeignetes Regenerationsgefäß überführt werden. Die schonende Regenerierung des Kationenaustauschers kann beispielsweise als Batchregeneration in dem Fachmann bekannten Apparaten, z.B. gerührten Behältern, durchgeführt werden. Um den Kationenaustauscher nicht mechanisch zu schädigen, sind beispielsweise schonende, scherarme Rühr- und Förderaggregate vorteilhaft.

In einer bevorzugten Ausführungsform der Regeneration im Batchverfahren wird beispielsweise ein Kationenaustauscher im doppelten Volumen einer Mischung aus Phenol, Schwefelsäure und Wasser schonend und unter Stickstoff gerührt und das Katalysatorharz anschließend abfiltriert. Die Regenerationsprozedur kann bevorzugt mehrfach wiederholt werden, wobei die Zusammensetzung der Regenerationslösung, insbesondere der Wassergehalt variiert werden kann. Eine besonders bevorzugte Ausführung der Regeneration im Batchverfahren ist eine dreimalige Wiederholung der Regeneration, wobei der Wassergehalt der Regenerationslösung beispielsweise von 1 Gew.-% über 3 Gew.-% bis auf 5 Gew.-% gesteigert wird, während das Verhältnis Säure zu Phenol konstant gehalten wird.

Nach Überführung beispielsweise in eine Säule erfolgt die anschließende Neutralwäsche bevorzugt mit einem Gemisch aus Phenol und Wasser bis im Ablauf der Kationenaustauscherschüttung eine Leitfähigkeit < 50 µS/cm, bevorzugt < 20 µS/cm gemessen wird. Auch die Neutralwäsche kann Batchweise ausgeführt werden, wobei der Wassergehalt der Waschlösung variiert werden kann. Bevorzugt liegt der Wassergehalt der Neutralwäsche in der gleichen Größenordnung des Wassergehalts der Regenerationslösung. Die Neutralwäsche kann aber auch nach Überführung in eine Säule oder in den Reaktor kontinuierlich durchgeführt werden.

Die Entwässerung der Kationenaustauscherschüttung kann bevorzugt batchweise oder nach Überführung in eine Säule oder in den Reaktor auch kontinuierlich mit Phenol bis zu einem Restwassergehalt im Ablauf von bevorzugt 1 % erfolgen.

Die im erfindungsgemäßen Regenerierungsprozess bevorzugt verwendenden Säuren sind Brönstedt-Säuren (Protonen abgebende Säuren), bevorzugt Protonensäuren mit einem pKs-Wert von kleiner 3, wie z.B. Mineralsäuren oder starke organische Säuren. Besonders bevorzugt verwendete Säuren sind anorganische Säuren, wie z.B.: Schwefelsäure, Salzsäure, Salpetersäure, Phosphorsäure, Perchlorsäure, oder organische Säuren, wie z.B. aromatische Sulfonsäuren, halogenierte Carbonsäuren, wie z.B. Chloressigsäure oder Trifluoressigsäure, sehr acide Phenole, wie z.B. Pikrinsäure, oder ggf. auch Zitronensäure.

Ganz besonders bevorzugt sind Schwefelsäure, Salzsäure und Phosphorsäure. Die Konzentration dieser Säuren in Phenol sollte 20 mol-% bezogen auf die Mischung aus Phenol und Säure nicht übersteigen; bevorzugte Säurekonzentrationen in Phenol liegen bei 5 - 10 mol-%, besonders bevorzugt bei 3 - 5 mol-% bezogen auf das Gemisch.

Die für die Regenerierung eines deaktivierten Kationenaustauschers erforderliche Menge an Phenol-Säure-Mischung im Schritt a) beträgt mindestens ein halbes Bettvolumen (BV) bezogen auf das Ionenaustauscher-Katalysatorbett im Reaktor, maximal das vierfache BV, vorzugsweise aber ein bis zwei BV, wobei das BV als das Volumen definiert ist, das der Ionenaustauscher in der aktiven, entwässerten und Phenol-haltigen H-Form in einem zylindrischen Reaktor einnimmt. Befindet sich der Kationenaustauscher beispielsweise als Katalysatorbett in einem kontinuierlich betriebenen Rohrreaktor, so wird die Phenol-Säure-Mischung langsam durch das zu regenerierende Katalysatorbett mit einer Strömungsgeschwindigkeit von vorzugsweise 1/10 bis 2 BV pro Stunde geleitet, um eine ausreichende Reaktionszeit der Säure mit dem Kationenaustauscher sicher zu stellen.

Bei einer Variante des erfindungsgemäßen Verfahrens wird die Durchströmung der Kationenaustauscherschüttung zeitweise angehalten, um die Reaktionszeit der Regenerationslösung mit den Katalysator deaktivierenden und zu entfernenden Komponenten zu verlängern. Die bevorzugte zusätzliche Reaktionszeit beträgt weniger als 24h, besonders bevorzugt 12 h und weniger.

Die Temperatur im Katalysatorbett des Reaktors während der Regenerierung des Kationenaustauschers sollte 90°C nicht übersteigen, um unerwünschte Nebenreaktionen zu vermeiden, sie sollte auch nicht unter 45°C liegen, um eine hinreichende Reaktionsgeschwindigkeit zu ermöglichen. Die bevorzugten Temperaturen gehen von 55°C bis 75°C.

Die Zugabe von Wasser zur sauren Spüllösung erfolgt gradientenweise, um eine zu hohe Wasserkonzentration in der Spüllösung zu vermeiden. Dies ist für eine erfolgreiche Regenerierung des Ionenaustauscherharzes von großer Bedeutung, da im Falle zu hoher Wasserkonzentrationen die Gefahr besteht, dass der osmotische Druck im Ionenaustauscherkorn so stark ansteigt, dass das mit den "fouling"-Komponenten belastete, starre und unelastische Korn zerreißt oder bricht. Dadurch werden Bruchstücke und Feinanteile des Ionenaustauscherkorns in der Größenordnung von µm gebildet, die nur aufwändig filtrierbar sind und dann im Normalbetrieb des Kationenaustauschers als Katalysator auf diese Weise in nachfolgende Prozessschritte gelangen können, in denen sie massive Probleme durch unerwünschte Nebenreaktionen verursachen können. Solche Bruchstücke können im Normalbetrieb des Kationenaustauschers als Katalysator auch zur Verstopfung von Filtern und damit zur Beeinträchtigung des BPA-Herstellverfahrens führen.

Durch die Kornbruch bedingte Veränderung der Gesamtkorngrößenverteilung des Kationenaustauschers kann ferner die Druckverlustcharakteristik in der durchströmten Kationenaustauscherschüttung negativ beeinflusst werden, was zu höherem Druckverlust in der Schüttung und möglichen hydraulischen Limitierungen und somit Produktionsmengenlimitierungen führen kann.

Die gradientenweise Zugabe von Wasser erfolgt erfindungsgemäß in der Weise, dass nach der Beendigung der Spülung des Kationenaustauschers mit der Phenol-Säure-Mischung in einem ersten nachfolgenden Schritt die Konzentration von Wasser in dieser sauren phenolischen Spüllösung von 0 auf 1 Gew.-% angehoben wird. Mit einer solchen, 0 - 1 Gew.-% Wasser enthaltenden sauren phenolischen Lösung wird der Kationenaustauscher mit einer Menge von 0,25 bis 4 BV dieser Lösung gespült. Vorzugsweise erfolgt diese Spülung mit einer Strömungsgeschwindigkeit durch das Katalysatorbett mit 1/10 bis 4 BV pro Stunde. Anschließend erfolgt in einem zweiten Schritt eine gradientenweise Erhöhung der Wasserkonzentration in der sauren phenolischen Spüllösung von 1 Gew.-% auf 5 Gew.-%. Mit einer solchen, 1 - 5 Gew.-% Wasser enthaltenden sauren phenolischen Lösung wird der Kationenaustauscher ebenfalls mit einer Menge von ca. einem BV dieser Lösung gespült. Auch hier erfolgt diese Spülung vorzugsweise mit einer Strömungsgeschwindigkeit durch das Katalysatorbett von ¼ BV pro Stunde. Mit diesen Maßnahmen werden die maximal mögliche Aktivierung der Sulfonsäuregruppen und die maximal mögliche Zersetzung von höhermolekularen Kondensationsprodukten im Kationenaustauscher erreicht, ohne diesen dabei mechanisch zu schädigen.

Nach dieser gradientenweisen Erhöhung der Wasserkonzentration in der sauren phenolischen Spüllösung müssen überschüssige Säure und Reste von Spaltprodukten aus dem Polymernetzwerk des Kationenaustauschers möglichst vollständig herausgewaschen werden, da sie anderenfalls im bestimmungsgemäßen Betrieb des Kationenaustauschers als Katalysator stören würden. Dies geschieht durch Waschen des Kationenaustauschers mit reinem Wasser oder mit einer Lösung von Phenol in Wasser. Typische Waschlösungen enthalten 5 - 25 Gew.-% Wasser in Phenol. Die bevorzugte Strömungsgeschwindigkeit liegt auch hier bei ¼ - 2 BV pro Stunde. Die Gesamtmenge der benötigten Spüllösung richtet sich nach der elektrischen Leitfähigkeit (µS/cm) der aus dem Katalysatorbett ablaufenden Spüllösung. Sie ist ein Maß für ionische Verunreinigungen im Wasser. Die Messmethoden sind dem Fachmann hinreichend bekannt. Diese Spülung mit Wasser oder Phenol /Wasser sollte so lange fortgesetzt werden, bis die elektrische Leitfähigkeit unter 50 µS/cm, bevorzugt unter 20 µS/cm sinkt. Die Temperatur des mit Wasser oder Phenol / Wasser gefluteten Katalysatorbetts sollte 80 °C nicht überschreiten und 45 °C nicht unterschreiten; bevorzugt sind Waschtemperaturen im Bereich von 55 °C bis 70 °C. Das insbesondere bei dieser Spülung verwendete Wasser sollte bevorzugt ein vollständig entsalztes Wasser sein.

Durch einen höheren Wassergehalt in der Neutralwäsche wird die Waschmenge reduziert, das Risiko der Schädigung des Ionenaustauschers aber erhöht. Deshalb ist hier eine hohe Vorsicht geboten. Weiterhin reduziert ein höherer Spüldurchsatz den Zeitbedarf der Regeneration. In beiden Fällen steht der Reaktor schneller wieder für die Produktion zur Verfügung.

In einer bevorzugten Ausführungsform wird der so regenerierte und gereinigte Kationenaustauscher zur Verwendung als Katalysator in der Bisphenolsynthese aktiviert, indem ihm beispielsweise das Wasser weitgehend entzogen wird. Diese Entwässerung erfolgt beispielsweise durch Spülen des Katalysatorbetts mit reinem Phenol solange, bis der Wassergehalt im Ablauf der rein phenolischen Spüllösung aus dem Katalysatorbett weniger als 1 Gew% Wasser beträgt.

Nach dieser Konditionierung steht der Kationenaustauscher als Katalysator beispielsweise für Bisphenolsynthesen aus Ketonen und Phenolen wieder zur Verfügung. Die Aktivität eines so regenerierten Kationenaustauschers als Katalysator ist so, dass das Aktivitätsniveau des neuen Katalysators nahezu vollständig wiederhergestellt wird. Der nach dem erfindungsgemäßen Verfahren regenerierte Kationenaustauscher zeigt im technischen Produktionsverfahren eine Katalysatordeaktivierungsrate, die der des neuen Katalysators entspricht.

Durch ggf. mehrfache Wiederholung des erfindungsgemäßen Verfahrens der Katalysatorregeneration lässt sich die Totalkapazität des Kationenaustauschers geringfügig steigern. Ein weiterer positiver Einfluss auf die Katalysatoraktivität ist jedoch kaum feststellbar.

Die erfindungsgemäße Regenerierung erfolgt bevorzugt an Ionenaustauscher in Festbettreaktoren für Kondensationsreaktionen, besonders bevorzugt für die Herstellung von Bis(4-hydroxyaryl)alkanen, ganz besonders bevorzugt für die Herstellung von 2,2-Bis-(4-hydroxyphenyl)-propan (Bisphenol A).

Geeignete Bis(4-hydroxyaryl)alkane, welche unter Verwendung des Ionenaustauschers als Katalysator erhältlich sind, sind beispielsweise solche der allgemeinen Formel (I), 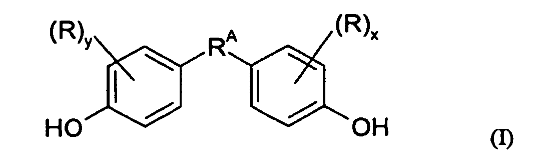 worin
- R^{A}: für einen linearen oder verzweigten C₁-C₁₈-Alkylenrest, bevorzugt C₁-C₆- Alkylenrest, oder einen C₅-C₁₈-Cycloalkylenrest, bevorzugt einen C₅-C₁₂- Cycloalkylenrest steht,
- R: unabhängig voneinander für einen linearen oder verzweigten C₁-C₁₈-Alkylrest, bevorzugt C₁-C₆-Alkylrest, einen C₅-C₁₈-Cycloalkylrest, bevorzugt einen C₅-C₁₂- Cycloalkylrest, einen C₆-C₂₄-Arylrest, bevorzugt einen C₆-C₁₂-Arylrest, oder einen Halogenrest stehen und
- x und y: unabhängig voneinander für 0 oder eine ganze Zahl von 1 bis 4, bevorzugt unab- hängig voneinander für 0, 1 oder 2 stehen.

Bevorzugte Bis(4-hydroxyaryl)alkane sind 2,2-Bis-(4-hydroxyphenyl)propan (Bisphenol A (BPA)), 2,4-Bis-(4-hydroxyphenyl)-2-methylbutan, 2,2-Bis-(3-methyl-4-hydroxyphenyl)-propan, Bis-(3,5-dimethyl-4-hydroxyphenyl)-methan, 2,2-Bis-(3,5-dimethyl-4-hydroxyphenyl)-propan, 2,4-Bis-(3,5-dimethyl-4-hydroxyphenyl)-2-methylbutan, 1,1-Bis-(4-hydroxyphenyl)-cyclohexan und 1,1-Bis-(4-hydroxyphenyl)-3,3,5-trimethylcyclohexan (Bisphenol TMC).

Besonders bevorzugte Bis(4-hydroxyaryl)alkane sind 2,2-Bis-(4-hydroxyphenyl)-propan (Bisphenol A (BPA)), 2,2-Bis(3,5-dimethyl-4-hydroxyphenyl)-propan, 1,1-Bis-(4-hydroxyphenyl)-cyclohexan und 1,1-Bis-(4-hydroxyphenyl)-3,3,5-trimethylcyclohexan (Bisphenol TMC). Ganz besonders bevorzugt ist 2,2-Bis-(4-hydroxyphenyl)-propan (Bisphenol A).

Bis(4-hydroxyaryl)alkane sind nach dem Fachmann bekannten Verfahren erhältlich durch Umsetzung von aromatischen mono-Hydroxyverbindungen, die in p-Position nicht substituiert sind, mit Ketonen, die wenigstens eine aliphatische Gruppe an der Carbonylfunktion aufweisen, in einer Kondensationsreaktion. Dabei wird vorzugsweise als Zwischenprodukt ein Addukt des Bis(4-hydroxyaryl)alkans und der als Edukt eingesetzten aromatischen mono-Hydroxyverbindung erhalten, welches anschließend in das gewünschte Bis(4-hydroxyaryl)alkan und aromatische mono-Hydroxyverbindung getrennt wird.

Geeignete aromatische mono-Hydroxyverbindungen sind beispielsweise solche der allgemeinen Formel (II), 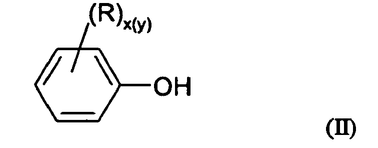 die in p-Position nicht substituiert sind und worin,
- R: unabhängig voneinander für einen linearen oder verzweigten C₁-C₁₈-Alkylrest, bevorzugt C₁-C₆-Alkylrest, einen C₅-C₁₈-Cycloalkylrest, bevorzugt einen C₅-C₁₂- Cycloalkylrest, einen C₆-C₂₄-Arylrest, bevorzugt einen C₆-C₁₂-Arylrest, oder einen Halogenrest stehen und
- x oder y: für 0 oder eine ganze Zahl von 1 bis 4, bevorzugt für 0, 1 oder 2 stehen.

Beispiele für geeignete aromatische mono-Hydroxyverbindungen der allgemeinen Formel (II) sind beispielsweise Phenol, o-und m-Kresol, 2,6-Dimethylphenol, o-tert.-Butylphenol, 2-Methyl-6-tert.-butylphenol, o-Cyclohexylphenol, o-Phenyl-phenol, o-Isopropylphenol, 2-Methyl-6-cyclopentylphenol, o-und m-Chlorphenol oder 2,3,6-Trimethylphenol. Bevorzugt sind Phenol, o-und m-Kresol, 2, 6-Dimethylphenol, o-tert.-Butylphenol und o-Phenyl-phenol, ganz besonders bevorzugt ist Phenol.

Geeignete Ketone sind beispielsweise solche der allgemeinen Formel (III), 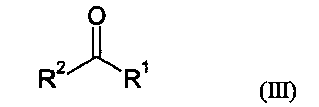 worin
- R¹: für einen linearen oder verzweigten C₁-C₁₈-Alkylrest, bevorzugt C₁-C₆-Alkylrest, steht und
- R²: für einen linearen oder verzweigten C₁-C₁₈-Alkylrest, bevorzugt C₁-C₆-Alkylrest, oder ei- nen C₆-C₂₄-Arylrest, bevorzugt einen C₆-C₁₂-Arylrest, steht oder
- R¹ und R²: zusammen für einen linearen oder verzweigten C₄-C₁₈-Alkylenrest, bevorzugt C₄-C₁₂- Alkylenrest stehen.

Beispiele für geeignete Ketone der allgemeinen Formel (III) sind Aceton, Methylethylketon, Methylpropylketon, Methylisopropylketon, Diethylketon, Acetophenon, Cyclohexanon, Cyclopentanon, Methyl-, Dimethyl- und Trimethylcyclohexanonen, die auch geminale Methylgruppen aufweisen können, z.B. 3,3-Dimethyl-5-methylcyclohexanon (Hydroisophoron). Bevorzugte Ketone sind Aceton, Acetophenon, Cyclohexanon und dessen Methylgruppen tragende Homologe, besonders bevorzugt ist Aceton.

C₁-C₆-Alkyl steht beispielsweise für Methyl, Ethyl, n-Propyl, iso-Propyl, n-Butyl, sec.-Butyl, tert.-Butyl, n-Pentyl, 1-Methylbutyl, 2-Methylbutyl, 3-Methylbutyl, neo-Pentyl, 1-Ethylpropyl, Cyclohexyl, Cyclopentyl, n-Hexyl, 1,1-Dimethylpropyl, 1,2-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl, 1-Ethyl-2-methylpropyl oder 1-Ethyl-2-methylpropyl, C₁-C₁₈-Alkyl darüber hinaus beispielsweise für n-Heptyl und n-Octyl, Pinakyl, Adamantyl, die isomeren Menthyle, n-Nonyl, n-Decyl, n-Dodecyl, n-Tridecyl, n-Tetradecyl, n-Hexadecyl, n-Octadecyl oder Stearyl.

C₁-C₆-Alkylen bzw. C₁-C₁₈-Alkylen steht beispielsweise für die den vorangehenden Alkylgruppen entsprechenden Alkylengruppen.

C₅-C₁₂-Cycloalkyl steht beispielsweise für Cylopentyl, Cyclohexyl, Cyclooctyl oder Cyclododecyl.

Beispiele für C₆-C₂₄-Aryl oder C₆-C₁₂-Aryl sind Phenyl, o-, p-, m-Tolyl, Naphthyl, Phenanthrenyl, Anthracenyl oder Fluorenyl.

**Halogen** kann für Fluor, Chlor, Brom oder Iod, bevorzugt für Fluor oder Chlor, besonders bevorzugt für Chlor stehen.

### Beispiele:

Die Katalysatorregeneration wurde anhand dreier Kriterien bestimmt:
   - Katalysatoraktivität
   - Totalkapazität des Kationenaustauschers
   - Kornbruchanteil vor und nach der Regeneration

### Bestimmung der Katalysatoraktivität:

Die Katalysatoraktivität wurde anhand des Acetonumsatzes in einer kontinuierlich durchströmten Kationenaustauscherschüttung bei einer Temperatur von 60 °C und Verwendung eines Cokatalysators, einem Mercaptanderivat, bestimmt.

### Bestimmung der Totalkapazität:

Die Totalkapazität des Kationenaustauschers wurde nach DIN 54403 bestimmt.

### Bestimmung des Kornbruchanteils:

Der Kornbruchanteil wurde durch computerunterstützte statistische Auswertung lichtmikroskopischer Aufnahmen bestimmt.

### Beispiel 1

280 g phenolfeuchter Kationenaustauscher wurden mit Hilfe von Phenol in die Säule gespült. "Phenolfeuchter Kationenaustauscher" bezeichnet einen Kationenaustauscher, dessen Wassergehalt durch Spülen mit Phenol reduziert wird, so dass im Ablauf der Phenolspülung weniger als 1 Gew.-% Wasser gemessen werden. Anschließend wurde das überstehende Phenol abgelassen und die Säule auf 70 °C temperiert und die Kationenaustauscherschüttung mit ca. 1 BV einer Mischung aus 5 % Schwefelsäure in Phenol (95 %) beaufschlagt, so dass die Kationenaustauscherschüttung komplett mit der flüssigen Mischung bedeckt ist. Die Kationenaustauscherschüttung wurde mit einem Gemisch aus 270 g Phenol und 15 g konzentrierter Schwefelsäure mit einem Volumenstrom von ca. ¼ BV pro Stunde beaufschlagt, wobei der Wasseranteil der phenolischen Schwefelsäure innerhalb der 4 h von 0 Gew.-% auf 1 Gew.-% linear erhöht wurde. Anschließend wurde die Kationenaustauscherschüttung mit einem Gemisch aus 270 g Phenol, 3 g Wasser und 15 g konzentrierter Schwefelsäure mit einem Volumenstrom von ca. ¼ BV pro Stunde beaufschlagt, wobei der Wasseranteil der phenolischen Schwefelsäure innerhalb der nächsten 3 h von 1 Gew.-% auf 5 Gew.-% linear erhöht wurde.

Die anschließende Neutralwäsche erfolgte mit einem Gemisch aus 95 Teilen Phenol und 5 Teilen Wasser mit einem Volumenstrom von 0,7 BV pro Stunde, wobei der Wasseranteil der Waschlösung innerhalb von 5 h auf 3 Teile Phenol und 1 Teil Wasser erhöht wurde, und anschließend so lange gespült wurde, bis im Ablauf der Kationenaustauscherschüttung eine Leitfähigkeit < 20 µS/cm gemessen wurde. Die Entwässerung der Kationenaustauscherschüttung erfolgte mit Phenol bis zu einem Restwassergehalt im Ablauf von 1 %.

### Beispiel 2

280 g phenolfeuchter Kationenaustauscher wurden mit Hilfe von Phenol in die Säule gespült. Anschließend wurde das überstehende Phenol abgelassen und die Säule auf 70 °C temperiert und die Kationenaustauscherschüttung mit ca. 1 BV einer Mischung aus 5 % Schwefelsäure in Phenol (95 %) beaufschlagt, so dass die Kationenaustauscherschüttung komplett mit der flüssigen Mischung bedeckt ist. Die Kationenaustauscherschüttung wurde mit einem Gemisch aus 270 g Phenol und 15 g konzentrierter Schwefelsäure mit einem Volumenstrom von ca. ¼ BV pro Stunde beaufschlagt, wobei der Wasseranteil der phenolischen Schwefelsäure innerhalb der 4 h von 0 Gew.-% auf 1 Gew.-% erhöht wurde. Anschließend wurde die Kationenaustauscherschüttung mit einem Gemisch aus 270 g Phenol, 3 g Wasser und 15 g konzentrierter Schwefelsäure mit einem Volumenstrom von ca. ¼ BV pro Stunde beaufschlagt, wobei der Wasseranteil der phenolischen Schwefelsäure innerhalb der nächsten 3 h von 1 Gew.-% auf 5 Gew.-% erhöht wurde.

Die anschließende Neutralwäsche erfolgte mit einem Gemisch aus 95 Teilen Phenol und 5 Teilen Wasser mit einem Volumenstrom von 0,7 BV pro Stunde bis im Ablauf der Kationenaustauscherschüttung eine Leitfähigkeit < 20 µS/cm gemessen wurde. Die Entwässerung der Kationenaustauscherschüttung erfolgte mit Phenol bis zu einem Restwassergehalt im Ablauf von 1 %.

### Vergleichsbeispiel 3

280 g phenolfeuchter Kationenaustauscher wurden mit Hilfe von Phenol in die Säule gespült. Anschließend wurde das überstehende Phenol abgelassen und die Säule auf 70 °C temperiert. Die Kationenaustauscherschüttung wurde mit einem Gemisch aus 540 g Phenol, 30 g Wasser und 30 g konzentrierter Schwefelsäure mit einem Volumenstrom von ¼ BV pro Stunde beaufschlagt. Die anschließende Neutralwäsche erfolgte mit einem Gemisch aus 3 Teilen Phenol und 1 Teil Wasser mit einem Volumenstrom von 0,7 BV pro Stunde bis im Ablauf der Kationenaustauscherschüttung eine Leitfähigkeit < 20 µS/cm gemessen wurde. Die Entwässerung der Kationenaustauscherschüttung erfolgte mit Phenol bis zu einem Restwassergehalt im Ablauf von 1 %.

### Vergleichsbeispiel 4

280 g phenolfeuchter Kationenaustauscher wurden mit Hilfe von Phenol in die Säule gespült. Anschließend wurde das überstehende Phenol abgelassen und die Säule auf 70 °C temperiert. Die Kationenaustauscherschüttung wurde jeweils drei mal mit einem Gemisch aus 540 g Phenol, 30 g Wasser und 30 g konzentrierter Schwefelsäure mit einem Volumenstrom von ¼ BV pro Stunde beaufschlagt.

Die anschließende Neutralwäsche erfolgte mit einem Gemisch aus 3 Teilen Phenol und 1 Teil Wasser mit einem Volumenstrom von 0,7 BV pro Stunde bis im Ablauf der Kationenaustauscherschüttung eine Leitfähigkeit < 20 µS/cm gemessen wurde. Die Entwässerung der Kationenaustauscherschüttung erfolgte mit Phenol bis zu einem Restwassergehalt im Ablauf von 1 %.

### Vergleichsbeispiel 5

280 g phenolfeuchter Kationenaustauscher wurden mit Hilfe von Phenol in die Säule gespült. Anschließend wurde das überstehende Phenol abgelassen und die Säule auf 70 °C temperiert. Die Kationenaustauscherschüttung wurde mit einem Gemisch aus 570 g Phenol und 30 g konzentrierter Schwefelsäure mit einem Volumenstrom von ¼ BV pro Stunde beaufschlagt.

Die anschließende Neutralwäsche erfolgte mit einem Gemisch aus 96 Teilen Phenol und 4 Teilen Wasser mit einem Volumenstrom von 0,7 BV pro Stunde bis im Ablauf der Kationenaustauscherschüttung eine Leitfähigkeit < 20 µS/cm gemessen wurde. Die Entwässerung der Kationenaustauscherschüttung erfolgte mit Phenol bis zu einem Restwassergehalt im Ablauf von 1 %.

### Vergleichsbeispiel 6

280 g phenolfeuchter Kationenaustauscher wurden mit Hilfe von Phenol in die Säule gespült. Anschließend wurde das überstehende Phenol abgelassen und die Säule auf 70 °C temperiert. Die Kationenaustauscherschüttung wurde mit einem Gemisch aus 540 g Phenol, 30 g Wasser und 30 g konzentrierter Schwefelsäure mit einem Volumenstrom von ¼ BV pro Stunde beaufschlagt.

Die anschließende Neutralwäsche erfolgte mit einem Gemisch aus 3 Teilen Phenol und 1 Teil Wasser mit einem Volumenstrom von 0,7 BV pro Stunde bis im Ablauf der Kationenaustauscherschüttung eine Leitfähigkeit < 20 µS/cm gemessen wurde. Die Entwässerung der Kationenaustauscherschüttung erfolgte mit Phenol bis zu einem Restwassergehalt im Ablauf von 1 %.

### Vergleichsbeispiel 7

280 g phenolfeuchter Kationenaustauscher wurden mit Hilfe von Phenol in die Säule gespült. Anschließend wurde das überstehende Phenol abgelassen und die Säule auf 70 °C temperiert. Die Kationenaustauscherschüttung wurde mit einem Gemisch aus 540 g Phenol, 30 g Wasser und 30 g konzentrierter Schwefelsäure mit einem Volumenstrom von ¼ BV pro Stunde beaufschlagt.

Die anschließende Neutralwäsche erfolgte mit einem Gemisch aus 95 Teilen Phenol und 5 Teilen Wasser mit einem Volumenstrom von 0,7 BV pro Stunde bis im Ablauf der Kationenaustauscherschüttung eine Leitfähigkeit < 20 µS/cm gemessen wurde. Die Entwässerung der Kationenaustauscherschüttung erfolgte mit Phenol bis zu einem Restwassergehalt im Ablauf von 1 %.

### Vergleichsbeispiel 8

280 g phenolfeuchter Kationenaustauscher wurden mit Hilfe von Phenol in die Säule gespült. Anschließend wurde das Phenol komplett abgelassen, die Kationenaustauscherschüttung mit einem Gemisch aus 270 g Phenol, 15 g Wasser und 15 g konzentrierter Schwefelsäure beaufschlagt und die Säule auf 70 °C temperiert. Anschließend wurde die Kationenaustauscherschüttung mit einem Gemisch aus 270 g Phenol, 15 g Wasser und 15 g konzentrierter Schwefelsäure mit einem Volumenstrom von ¼ BV pro Stunde beaufschlagt.
Die anschließende Neutralwäsche erfolgte mit einem Gemisch aus 95 Teilen Phenol und 5 Teilen Wasser mit einem Volumenstrom von 0,7 BV pro Stunde bis im Ablauf der Kationenaustauscherschüttung eine Leitfähigkeit < 20 µS/cm gemessen wurde. Die Entwässerung der Kationenaustauscherschüttung erfolgte mit Phenol bis zu einem Restwassergehalt im Ablauf von 1 %.

### Beispiel 9

Der nach dem erfindungsgemäßen Verfahren nach Beispiel 1 regenerierte Kationenaustauscher wurde in einem Langzeittest von über 3600 h bei einer Temperatur von 60 °C in einer durchströmten Kationenaustauscherschüttung getestet. Die festgestellte geringe Deaktivierungsrate entspricht der des neuen Kationenaustauschers.

| Beispiel | Acetonumsatz vor Regeneration | Acetonumsatz nach Regeneration | Totalkapazität vor Regeneration | Totalkapazität nach Regeneration | Delta Bruchanteil | Gesamtzeit der Regeneration |
|---|---|---|---|---|---|---|
| | [%] | [%] | [mol / kg] | [mol/kg] | [%] | [h] |
| 1 | 62,7 | 84,5 | 3,8 | 4,38 | 27 | 41 |
| 2 | 62,7 | 84,3 | 3,8 | 4,35 | 23 | 47 |
| 3* | 62,7 | 84,8 | 3,8 | 4,4 | 47 | 43 |
| 4* | 62,7 | 84,0 | 3,8 | 4,4 | | 59 |
| 5* | 58,4 | 66,9 | 3,28 | 3,64 | | 54 |
| 6* | | | | | 47 | 43 |
| 7* | | | | | 34,5 | 48 |
| 8* | 62,7 | 83,7 | 3,2 | 4,3 | | 43 |

| | | | | | | |
|---|---|---|---|---|---|---|
| * Vergleichsbeispiel | | | | | | |

## Patentansprüche

1. Regenerierungsverfahren für deaktivierte oder partiell deaktivierte saure Kationenaustauscher durch Spülen eines außer Betrieb genommenen Ionenaustauscher-Katalysators mit einer Mischung aus Phenol und Säure, wobei der Wassergehalt gradientenweise in der zur Spülung des Ionenaustauscher-Katalysator verwendeten Phenol-Säure-Mischung erhöht wird, wobei das Regenerierungsverfahren wenigstens folgende Schritte umfasst:
a) Spülen eines außer Betrieb genommenen Ionenaustauscher-Katalysators mit einer Mischung aus Phenol und Säure, wobei der Gehalt an Säure in der Phenol-Säure-Mischung kleiner 10 Gew.-% und größer 0 Gew.-% ist, die Temperatur in dem mit der Phenol-Säure-Mischung gefluteten Reaktorbett geht dabei von 45°C bis 90°C, die Menge an Phenol-Säure-Mischung beträgt 1 bis 4 Bettvolumina (BV), entsprechend dem Ionenaustauscher-Katalysatorbett im Reaktor
b) gradientenweise Erhöhung des Wassergehaltes in der zur Spülung des Ionenaustauscher-Katalysator verwendeten Phenol-Säure-Mischung von 0 Gew.-% Wasser auf 1 Gew.-% Wasser, wobei das Gesamtvolumen dieser Mischung als Spüllösung 0,25 bis 4 BV beträgt und gradientenweise Erhöhung des Wassergehaltes in der zur Spülung des Ionenaustauscher-Katalysator verwendeten Phenol-Säure-Mischung von 1 Gew.-% Wasser auf 5 Gew.-% Wasser, wobei das Gesamtvolumen dieser Mischung als Spüllösung 0,25 bis 4 BV beträgt
c) Spülen des Ionenaustauscher-Katalysators mit einer Mischung aus Phenol und Wasser, die 5 - 25 Gew.-% Wasser enthält, bis die elektrische Leitfähigkeit der Spüllösung kleiner 50 µS/cm
d) Entwässern des so gespülten Ionenaustauscher-Katalysators mit reinem Phenol bis der Restwassergehalt in dem Spül-Phenol kleiner als 1 Gew.-% ist.

2. Verfahren gemäß Anspruch 1, **dadurch gekennzeichnet, dass** in Schritt a) der Gehalt an Säure in der Phenol-Säure-Mischung von 1 bis 5 Gew.-% ist.

3. Verfahren gemäß Anspruch 1 oder 2, **dadurch gekennzeichnet, dass** in Schritt a) die Temperatur in dem mit der Phenol-Säure-Mischung gefluteten Reaktorbett von 60°C bis 70°C geht.

4. Verfahren gemäß einem der Ansprüche 1 bis 3, **dadurch gekennzeichnet, dass** in Schritt a) die Menge an Phenol-Säure-Mischung 2 Bettvolumen beträgt.

5. Verfahren gemäß einem der Ansprüche 1 bis 4, **dadurch gekennzeichnet, dass** in Schritt c) das Spülen des Ionenaustauscher-Katalysators erfolgt, bis die elektrische Leitfähigkeit der Spüllösung kleiner 20 µS/cm ist.

6. Verfahren gemäß einem der voranstehenden Ansprüche, **dadurch gekennzeichnet, dass** der Regenerationsprozess im Reaktor stattfindet.

7. Verfahren gemäß einem der voranstehenden Ansprüche, **dadurch gekennzeichnet, dass** eine oder mehrere Brönstedt-Säuren verwendet werden, mit einem pKs-Wert von kleiner 3.

8. Verfahren gemäß Anspruch 7, **dadurch gekennzeichnet, dass** Schwefelsäure, Salzsäure, Salpetersäure, Phosphorsäure, Perchlorsäure, oder aromatische Sulfonsäuren, halogenierte Carbonsäuren, Chloressigsäure oder Trifluoressigsäure, Pikrinsäure, oder Zitronensäure oder Mischungen daraus verwendet werden.

9. Verfahren gemäß einem der voranstehenden Ansprüche, **dadurch gekennzeichnet, dass** die Säuren in Phenol nicht 20 mol-% bezogen auf die Mischung aus Phenol und Säure übersteigen.

10. Verfahren gemäß Anspruch 9, **dadurch gekennzeichnet, dass** die Säurekonzentrationen in Phenol bei 5 bis 10 mol-% bezogen auf das Gemisch liegen.

11. Verfahren gemäß Anspruch 10, **dadurch gekennzeichnet, dass** die Säurekonzentrationen bei 1 bis 5 mol-% bezogen auf das Gemisch liegen.

12. Verfahren gemäß einem der voranstehenden Ansprüche, **dadurch gekennzeichnet, dass** der zu regenerierenden Katalysator zur Bisphenolsynthese benützt wurde.

13. Verfahren gemäß einem der voranstehenden Ansprüche, **dadurch gekennzeichnet, dass** der regenerierte Katalysator in der Bisphenolsynthese eingesetzt wird.

## Claims

1. Regeneration method for deactivated or partly deactivated acidic cation exchangers by washing an ion exchange catalyst taken out of operation with a mixture of phenol and acid, the water content being increased by the gradient method in the phenol-acid mixture used for washing the ion exchange catalyst, the regeneration method comprising at least the following steps:
a) washing an ion exchange catalyst taken out of operation with a mixture of phenol and acid, the content of acid in the phenol-acid mixture being less than 10% by weight and greater than 0% by weight, the temperature in the reactor bed flooded with the phenol-acid mixture going from 45°C to 90°C, the amount of phenol-acid mixture being 1 to 4 bed volumes (BV), corresponding to the ion exchange catalyst bed in the reactor
b) increasing the water content by the gradient method in the phenol-acid mixture used for washing the ion exchange catalyst from 0% by weight of water to 1% by weight of water, the total volume of this mixture as wash solution being 0.25 to 4 BV, and increasing the water content by the gradient method in the phenol-acid mixture used for washing the ion exchange catalyst from 1% by weight of water to 5% by weight of water, the total volume of this mixture as wash solution being 0.25 to 4 BV
c) washing the ion exchange catalyst with a mixture of phenol and water which contains 5 - 25% by weight of water, until the electrical conductivity of the wash solution is less than 50 µS/cm
d) dewatering the correspondingly washed ion exchange catalyst with pure phenol until the residual water content in the wash phenol is less than 1% by weight.

2. Method according to Claim 1, **characterized in that**, in step a), the content of acid in the phenol-acid mixture is from 1 to 5% by weight.

3. Method according to Claim 1 or 2, **characterized in that**, in step a), the temperature in the reactor bed flooded with the phenol-acid mixture is from 60°C to 70°C.

4. Method according to any of Claims 1 to 3, **characterized in that**, in step a), the amount of phenol-acid mixture is 2 bed volumes.

5. Method according to any of Claims 1 to 4, **characterized in that**, in step c), the washing of the ion exchange catalyst proceeds until the electrical conductivity of the wash solution is less than 20 µS/cm.

6. Method according to any of the preceding claims, **characterized in that** the regeneration process takes place in the reactor.

7. Method according to any of the preceding claims, **characterized in that** one or more Brönstedt acids having a pKa of less than 3 are used.

8. Method according to Claim 7, **characterized in that** sulphuric acid, hydrochloric acid, nitric acid, phosphoric acid, perchloric acid, or aromatic sulphonic acids, halogenated carboxylic acids, chloroacetic acid or trifluoroacetic acid, picric acid, or citric acid or mixtures thereof are used.

9. Method according to any of the preceding claims, **characterized in that** the acids in phenol do not exceed 20 mol%, based on the mixture of phenol and acid.

10. Method according to Claim 9, **characterized in that** the acid concentrations in phenol are 5 to 10 mol%, based on the mixture.

11. Method according to Claim 10, **characterized in that** the acid concentrations are 1 to 5 mol%, based on the mixture.

12. Method according to any of the preceding claims, **characterized in that** the catalyst to be regenerated was used for the bisphenol synthesis.

13. Method according to any of the preceding claims, **characterized in that** the regenerated catalyst is used in the bisphenol synthesis.

## Revendications

1. Procédé de régénération d'échangeurs de cations acides désactivés ou partiellement désactivés par rinçage d'un catalyseur à échange d'ions mis hors service avec un mélange de phénol et d'un acide, la teneur en eau étant augmentée graduellement dans le mélange phénol-acide utilisé pour le rinçage du catalyseur à échange d'ions, le procédé de régénération comprenant au moins les étapes suivantes :
a) le rinçage d'un catalyseur à échange d'ions mis hors service avec un mélange de phénol et d'un acide, la teneur en acide du mélange phénol-acide étant inférieure à 10 % en poids et supérieure à 0 % en poids, la température dans le lit de réacteur inondé avec le mélange phénol-acide étant de 45 °C à 90 °C, la quantité de mélange phénol-acide étant de 1 à 4 volumes de lit (BV), par rapport au lit de catalyseur à échange d'ions dans le réacteur,
b) l'augmentation graduelle de la teneur en eau du mélange phénol-acide utilisé pour le rinçage du catalyseur à échange d'ions de 0 % en poids d'eau à 1 % en poids d'eau, le volume total de ce mélange en tant que solution de rinçage étant de 0,25 à 4 BV, et l'augmentation graduelle de la teneur en eau du mélange phénol-acide utilisé pour le rinçage du catalyseur à échange d'ions de 1 % en poids d'eau à 5 % en poids d'eau, le volume total de ce mélange en tant que solution de rinçage étant de 0,25 à 4 BV,
c) le rinçage du catalyseur à échange d'ions avec un mélange de phénol et d'eau, qui contient 5 à 25 % en poids d'eau, jusqu'à ce que la conductivité électrique de la solution de rinçage soit inférieure à 50 µS/cm,
d) la déshydratation du catalyseur à échange d'ions ainsi rincé avec du phénol pur jusqu'à ce que la teneur en eau résiduelle du phénol de rinçage soit inférieure à 1 % en poids.

2. Procédé selon la revendication 1, **caractérisé en ce qu'**à l'étape a), la teneur en acide du mélange phénol-acide est de 1 à 5 % en poids.

3. Procédé selon la revendication 1 ou 2, **caractérisé en ce qu'**à l'étape a), la température dans le lit de réacteur inondé avec le mélange phénol-acide est de 60 °C à 70 °C.

4. Procédé selon l'une quelconque des revendications 1 à 3, **caractérisé en ce qu'**à l'étape a), la quantité de mélange phénol-acide est de 2 volumes de lit.

5. Procédé selon l'une quelconque des revendications 1 à 4, **caractérisé en ce qu'**à l'étape c), le rinçage du catalyseur à échange d'ions a lieu jusqu'à ce que la conductivité électrique de la solution de rinçage soit inférieure à 20 µS/cm.

6. Procédé selon l'une quelconque des revendications précédentes, **caractérisé en ce que** le procédé de régénération a lieu dans le réacteur.

7. Procédé selon l'une quelconque des revendications précédentes, **caractérisé en ce qu'**un ou plusieurs acides de Bronsted ayant une valeur de pKs inférieure à 3 sont utilisés.

8. Procédé selon la revendication 7, **caractérisé en ce que** l'acide sulfurique, l'acide chlorhydrique, l'acide nitrique, l'acide phosphorique, l'acide perchlorique ou des acides sulfoniques aromatiques, des acides carboxyliques halogénés, l'acide chloroacétique ou l'acide trifluoroacétique, l'acide picrique ou l'acide citrique ou leurs mélanges sont utilisés.

9. Procédé selon l'une quelconque des revendications précédentes, **caractérisé en ce que** les acides ne dépassent pas 20 % en moles du mélange phénol-acide dans le phénol.

10. Procédé selon la revendication 9, **caractérisé en ce que** les concentrations en acide dans le phénol sont de 5 à 10 % en moles par rapport au mélange.

11. Procédé selon la revendication 10, **caractérisé en ce que** les concentrations en acide sont de 1 à 5 % en moles par rapport au mélange.

12. Procédé selon l'une quelconque des revendications précédentes, **caractérisé en ce que** le catalyseur à régénérer a été utilisé pour la synthèse de bisphénol.

13. Procédé selon l'une quelconque des revendications précédentes, **caractérisé en ce que** le catalyseur régénéré est utilisé dans la synthèse de bisphénol.
